# EUROPÄISCHE PATENTANMELDUNG

(11) **EP 3 616 817 A1**
(43) Veröffentlichungstag der Anmeldung: **04.03.2020**
(21) Anmeldenummer: 18191662.8
(22) Anmeldetag: 30.08.2018
(51) Int. Cl.: B23D 75/00

(54) **VERFAHREN UND EINRICHTUNG ZUM ABRASIVEN BEARBEITEN EINER INNENSEITE EINES ROHRES, INSBESONDERE EINES ROHRHALBZEUGS FÜR EIN MEDIZINPRODUKT**

(71) Anmelder: BIOTRONIK AG, 8180 Bülach (CH)
(72) Erfinder: Bayer, Ullrich, 18209 Bad Doberan (DE); Dramm, Sarah, 18119 Rostock Diedrichshagen (DE); Jörn, Alexander, 18209 Bad Doberan (DE); Rüter, Björn, 18211 Ostseebad Nienhagen (DE); Homuth, Torsten, 18211 Ostseebad Nienhagen (DE); Fröhlich, Nils, 18057 Rostock (DE)
(74) Vertreter: Galander, Marcus

(57) **Zusammenfassung**

Die Erfindung betrifft ein Verfahren zur Bearbeitung einer Innenseite (2a) eines entlang einer Längsachse (x) erstreckten Rohres (2), wobei die Innenseite (2a) einen Innenraum (3) des Rohres (2) zugewandt ist, und wobei das Verfahren zumindest die folgenden Schritte aufweist: Einführen eines Werkzeugs (10) in den Innenraum (3) des Rohres (2), wobei das Werkzeug (1) eine Außenseite (10a) mit einer Vielzahl an zur Außenseite (10a) hin offenen Strömungskanälen (11) in Form von Rillen aufweist, die in einer Umfangsrichtung (U) des Werkzeugs (10) nebeneinander angeordnet sind, und wobei jede Rille (11) an der Außenseite (10a) jeweils zwei Schneidkanten (12) ausbildet, und Bewegen des Werkzeugs (10) im Innenraum (3) des Rohres (2) entlang der Längsachse (x) des Rohres (2) sowie gleichzeitiges Rotieren des Werkzeugs (10) in der Umfangrichtung (U) des Werkzeugs (10) durch Beaufschlagen des Werkzeugs (10) mit einem gasförmigen Medium und/oder durch Einwirken auf das Werkzeug (10) mit einem magnetischen Wechselfeld , wobei von der Innenseite (2a) des Rohres (2) abragende Verunreinigungen (20) des Rohres (2) mittels der Schneidkanten (12) abgetrennt werden. Weiterhin betrifft die Erfindung eine Einrichtung (1) zur Durchführung des Verfahrens.

## Beschreibung

Die vorliegende Erfindung betrifft ein Verfahren und Einrichtung zum abrasiven Bearbeiten einer Innenseite eines Rohres, insbesondere eines Rohrhalbzeugs für ein Medizinprodukt.

Rohre bzw. Rohrhalbzeuge, die als Ausgangsmaterial für Medizinprodukte zur Anwendung kommen, erfüllen regelmäßig nicht die Anforderungen hinsichtlich der geforderten Geometrien und der Freiheit an Oberflächenverunreinigungen. Derartige Oberflächenverunreinigungen können z. B. bei Herstellungsprozessen durch Reste von Schmierstoffen und anderer Verarbeitungshilfsmittel gebildet werden, sowie durch Ablagerungen von Werkzeugoberflächen auf den Rohrinnenoberflächen.

Vaskulare Gefäßimplantate werden in der Regel durch das Laserschneiden nahtlos gezogener Rohre hergestellt. Die Forderung nach nahtlos gezogenen Rohren ergibt sich aus der Tatsache, dass das Zusammenschweißen gebogener Bleche zu indifferenten Halbzeugeigenschaften - vor allem in der Schweißnaht - führt, die eine Anwendung für Stents verbietet. Dies ergibt sich vor allem aus den Forderungen zur Ermüdungsfestigkeit pulsatil beanspruchter Implantate.

Bei der Fertigung nahtlos gezogener Rohre kommen verschiedene Technologien zum Einsatz. Unabhängig davon ist es unvermeidlich, dass zum Einstellen der gewünschten Rohrdimensionen Umformwerkzeuge verwendet werden, die einen direkten Stoffkontakt zum Rohr haben und deshalb aufgrund der Kräfte bei der Umformung gewissen Verschleißerscheinungen unterliegen.

Dieser Verschleiß kann materialabtragenden (abrasiven) oder materialauftragenden (adhäsiven) Charakter oder Kombinationen von beiden annehmen. Es ist gängige Praxis, dass durch den Einsatz von Umformhilfsmitteln, wie Schmierstoffen, derartige Verschleißerscheinungen minimiert werden können. Dies birgt jedoch wiederum die Gefahr, dass Schmierstoffe mit den Rohroberflachen eine chemische Wechselwirkung entfalten. Diese Effekte werden durch thermische Prozesse (Warmumformung), die bei den meisten Rohrwerkstoffen (aufgrund materialbedingt begrenzter Kaltumformbarkeit) zur Anwendung kommen, verstärkt. Reaktionsprodukte aus den Werkzeugwerkstoffen (z. B. Stahl oder Hartmetall) und verschiedenartigsten, meist hoch kohlenstoffhaltigen Schmierstoffen verbinden sich mit den Rohroberflächen, und es kommt zur Bildung unterschiedlich harter an den Rohroberflächen haftender Auflagerungen/Verbindungen. Diese können unterschiedliche geometrische Formen (Einzelpartikel, langgestreckte Inseln, Plateaus) annehmen. In den meisten Fällen sind diese in Rohrlängsachse orientiert.

Um für die Weiterverarbeitung zu einem Medizinprodukt eine hinreichende Oberflächenqualität der Innenseite des betreffenden Rohres zu erzielen, ist es im Stand der Technik bekannt, Rohrhalbzeuge der eingangs genannten Art mittels Honen, Läppen, Druckfließläppen, Sandstrahlen, chemischer Abtragung mittels Beizlosungen, Spülen mit kohlenwasserstoffhaltigen Lösungen mit und ohne Ultraschallunterstützung oder mittels Innenbürsten zu bearbeiten.

Hierbei erweist sich jedoch das Honen bei Rohrlängen oberhalb von 1m als äußerst schwierig und aufwändig. Weiterhin besteht beim Läppen und Druckfließläppen die Gefahr des Verbleibs von Resten des Läppmittels im Rohr, was dann wiederum mit flüssigen Reinigungsmitteln entfernt werden muss. Weiterhin erfolgt beim Läppen bzw. Druckfließläppen der Abtrag vorzugsweise in Rohrlängsrichtung, d. h., bestehende, in Rohrlängsrichtung verlaufende grabenartige Riefen werden eher vertieft als geglättet.

Weiterhin erfolgt beim Sandstrahlen der Abtrag ebenfalls vorzugsweise in Rohrlängsrichtung, d. h., auch hier werden bestehende Riefen eher vertieft als geglättet. Ferner bleiben oftmals Reste des Strahlgutes auf der Rohrinnenoberfläche, verhaken sich dort und verbleiben teilweise form- und kraftschlüssig in den rauen Rohrinnenoberflachen, wobei ein komplettes Entfernen des Strahlgutes durch nachfolgende Prozesse (z. B. bei der Stentherstellung durch Beizen oder Elektropolieren) nicht garantiert ist. Ebenso ist nicht auszuschließen, dass durch den Sandstrahlprozess Verunreinigungen sogar weiter in die Rohroberfläche hineingedrückt werden.

Hinsichtlich des chemischen Abtrags mittels Beizlosungen ist über die Rohrlänge regelmäßig mit einer nicht einheitlichen und nachlassenden Beizwirkung zu rechnen, was zu unerwünschten Wanddickenunterschieden zwischen Rohranfang, Rohrmitte und Rohrende führen kann. Aufgrund der hohen chemischen Resistenz von üblichen Rohrmaterialien müssen hochwirksame und äußerst aggressive Beizlösungen zur Anwendung kommen, die zudem mit hohem Drücken durch das Rohr gedrückt werden. Dies führt zu hohen Aufwendungen beim Arbeitsschutz und bei der umweltgerechten Entsorgung der gebrauchten Beizlösungen.

Schließlich birgt das Innenbürsten den Hauptnachteil, dass die Durchzugskräfte, die durch die innere Reibung der Borsten an der Rohrinnenwand entstehen, bei langen Rohren sehr groß sind. Diese Reibung lässt sich nur durch die Verringerung der Differenz zwischen dem Bürstenaußendurchmesser und dem Rohrinnendurchmesser oder durch angepasste Bürstengeometrien minimieren. Beides führt zu einem unzureichenden Reinigungseffekt der Rohrinnenoberflächen.

Weiterhin wird in Junmo Kang et al., Procedia CIRP 1 (2012) 414 - 418, eine magnetische Schleifbearbeitung beschrieben, bei der mittels eines Mehrpolsystems unter Verwendung eines teilweise wärmebehandelten Magnetwerkzeugs das gleichzeitige Bearbeiten mehrerer Bereiche in Kapillarrohren möglich ist.

Ausgehend von dem oben dargestellten Stand der Technik liegt der vorliegenden Erfindung die Aufgabe zugrunde, ein Verfahren sowie eine Einrichtung zu schaffen, die es ermöglichen die der geforderten Rohrzusammensetzung nicht entsprechenden Fremdmaterialien rückstandsfrei zu entfernen. Insbesondere soll dabei ein besonderes Augenmerk darauf gelegt werden, dass die ursprüngliche Rohrgeometrie nicht negativ beeinflusst wird, wie es zum Beispiel der Fall ist, wenn sich Reinigungsmedien nur in Richtung der Rohrlängsachse entfalten.

Diese Aufgabe wird durch ein Verfahren mit den Merkmalen des Anspruchs 1 sowie durch eine Einrichtung mit den Merkmalen des Anspruchs 12 gelöst. Vorteilhafte Ausgestaltungen dieser Erfindungsaspekte sind in den Unteransprüchen angegeben und werden nachfolgend beschrieben.

Gemäß Anspruch 1 wird ein Verfahren zur Bearbeitung einer Innenseite eines entlang einer Längsachse erstreckten Rohres offenbart, wobei die Innenseite einem Innenraum des Rohres zugewandt ist bzw. diesen Innenraum begrenzt, und wobei das Verfahren zumindest die folgenden Schritte aufweist:
- Einführen eines Werkzeugs in den Innenraum des Rohres, wobei das Werkzeug eine der Innenseite des Rohres zugewandten Außenseite aufweist, in der eine Vielzahl an Strömungskanälen in Form von Rillen ausgebildet sind, die in einer Umfangsrichtung des Werkzeugs nebeneinander angeordnet sind, und wobei jede Rille an der Außenseite jeweils mindestens eine, bevorzugt zwei, Schneidkante(n) ausbildet, und
- Bewegen des Werkzeugs im Innenraum des Rohres entlang der Längsachse des Rohres sowie gleichzeitiges Rotieren des Werkzeugs in der Umfangrichtung des Werkzeugs durch Beaufschlagen des Werkzeugs mit einem Druck bzw. einem gasförmigen Medium und/oder durch Einwirken auf das Werkzeug mit einem magnetischen Wechselfeld , wobei von der Innenseite des Rohres abragende Verunreinigungen des Rohres mit Hilfe der Schneidkanten entfernt werden.

Das Werkzeug zeichnet sich insbesondere dadurch aus, dass es im Innenraum des zu bearbeitenden Rohres nicht mittels einer starren Kopplung, z. B. in Form einer Welle, bewegt wird, die das Werkzeug mit einem außerhalb des Werkzeuges angeordneten Aktuator mechanisch verbindet. Vielmehr ist lediglich das Werkzeug vollständig im Innenraum des Rohres angeordnet bzw. anordenbar und wird dort durch Druck- bzw. Unterdruckbeaufschlagung und/oder mittels eines magnetischen Wechselfeldes bewegt. Die abgetrennten Verunreinigungen bzw. Partikel können dabei insbesondere über die Strömungskanäle aus dem Innenraum des Rohres ausgestoßen werden, z. B. mittels des gasförmigen Mediums. Für den Fall, dass ein magnetisches Wechselfeld verwendet wird, um das Werkzeug zu bewegen/rotieren, kann z. B. eine entlang des Rohres verfahrbare Spule verwendet werden, in der zur Erzeugung des magnetischen Wechselfeldes ein Strom fließt. Die Spule kann dabei das Rohr umgreifen.

Das Wirkprinzip der vorliegenden Erfindung beruht also insbesondere auf einem Innenwerkzeug mit Schneidkanten sowie insbesondere einer harten, verschleißfesten und rauen Außenseite. Der Außendurchmesser des Werkzeugs wird vorzugsweise dem jeweiligen Innendurchmesser des zu bearbeitenden Rohres angepasst.

Um die gewünschten Innenbearbeitungseffekte zu erzielen, wird das Werkzeug insbesondere in die Lage versetzt, sich im Innenraum des Rohres sowohl radial als auch in Richtung der Längsachse des Rohres zu bewegen. Diese mehrachsige Bewegung kann mittels verschiedener Wirkprinzipien erfolgen. Dies gelingt zum einen mittels eines vorzugsweise alternierenden Unterdrucks oder Überdrucks und einer strömungstechnisch optimierten Oberflächenstrukturierung der Außenseite des Werkzeugs mittels der Strömungskanäle. Alternativ kann das Werkzeug auch durch ein magnetisches Feld in Bewegung gesetzt werden. Dies kann z. B. mit Hilfe einer außen um das Rohr geführten Spule erreicht werden Diese kann über die gesamte Rohrlange verschoben werden und das sich im Innenraum in einer taumelartigen Bewegung rotierende Werkzeug folgt dieser Bewegung. Der geforderte Abtrag der Innenseite des Rohres kann durch lokale Verweilzeiten, die Umdrehungsgeschwindigkeit des Werkzeugs in Umfangsrichtung des Werkzeugs und der Abrasionswirkung der Außenseite des Werkzeugs bzw. der Schneidkanten variiert werden.

Die Erfindung erlaubt somit insbesondere für die Stentherstellung (koronare, periphere oder TAVI-Stents) Rohrhalbzeuge zur Verfügung zu stellen, die zu einer wesentlich verringerten Fehlerquote hinsichtlich der Innenoberflächenbeschaffenheit führen. Insbesondere können Fremdmaterialien auf der Innenseite des Rohres restlos entfernt werden, wobei Ziehriefen vermieden werden beziehungsweise bereits aus dem Rohrherstellungsprozess herrührende Ziehriefen eingeebnet werden. Die Erfindung erlaubt somit eine Verbesserung der Langzeitermüdungseigenschaften von aus behandelten Rohren hergestellten Stents. Weiterhin kann der Nachbearbeitungsaufwand von lasergeschnittenen Stents verringert werden. Schließlich besteht aufgrund der Erfindung auch die Möglichkeit des Einbringens lokal veränderter/begrenzter Rohrinnendurchmesser.

Die Strömungskanäle auf der Außenseite des Werkzeuges sorgen bei dem erfindungsgemäßen Bearbeitungsverfahren durch eine Bewegung des Werkzeuges, bei der sich Bewegungskomponenten entlang der Längsachse des zu reinigenden Rohres mit Bewegungskomponenten entlang der Umfangsrichtung des zu reinigenden Rohres überlagern, insbesondere durch die Rotation des Werkzeuges um sich selbst. Dadurch werden die Ablagerungen im Innenraum des zu bearbeitende Rohres nicht nur entlang der Rohrachse abgetragen. Das Bewegungsmuster aus den überlagernden Bewegungskomponenten hat automatisch auch eine Abtragung in radialer Richtung zur Folge. Dadurch werden in Rohrlängsrichtung verlaufende Riefen vermieden und Ablagerungen viel zuverlässiger entfernt. Die beschriebene Überlagerung der axialen und radialen Bewegungskomponenten stellt sich dabei unabhängig davon ein, ob das Werkzeug mittels Druck- bzw. Unterdruckbeaufschlagung und/oder mittels eines magnetischen Wechselfeldes bewegt wird. Verantwortlich für das Bewegungsmuster sind die Strömungskanäle auf der Außenseite des Werkzeuges. Diese sind wie eingangs beschrieben ausgestaltet. Eine derartige Ausgestaltung sorgt für die Überlagerung der axialen und radialen Bewegung und insbesondere für die Eigenrotation des Werkzeuges.

Das Werkzeug wird bei dem Verfahren gemäß einer bevorzugten Ausführungsform so entlang der Längsachse bewegt und dabei in der Umfangsrichtung bzw. um eine Rotationsachse rotiert, dass es eine Taumelbewegung ausführt.

Hinsichtlich der Bewegung des Werkzeugs entlang der Längsachse des Rohres sowie der Rotation des Werkzeugs in der Umfangsrichtung ist vorzugsweise gemäß einer Ausführungsform des Verfahrens vorgesehen, dass die Strömungskanäle entlang der Außenseite des Werkzeugs jeweils einen gekrümmten Verlauf aufweisen. Hierdurch kann beim Durchströmen des gasförmigen Mediums durch die Strömungskanäle eine Rotation des Werkzeugs in der Umfangsrichtung des Werkzeugs sowie ein Vortrieb des Werkzeugs in Richtung der Längsachse erzeugt werden.

Gemäß einer Ausführungsform der Erfindung ist vorgesehen, dass das Werkzeug kugelförmig oder ellipsoidal, insbesondere als Rotationsellipsoid, ausgebildet ist. Hierbei kann das Werkzeug insbesondere eine Zylindersymmetrie bezüglich der oben erwähnten Rotationsachse aufweisen, um die das Werkzeug rotiert wird.

Weiterhin ist gemäß einer alternativen Ausführungsform des Verfahrens vorgesehen, dass das Werkzeug zylinderförmig ausgebildet ist, wobei insbesondere die besagte Rotationsachse, um die das Werkzeug rotiert wird, eine Zylinderachse des Werkzeugs ist. Aufgrund der Taumelbewegung des Werkzeugs wenn dieses hin- und herbewegt wird und dabei rotiert wird, variiert die Lage der Rotations- bzw. Zylinderachse des Werkzeugs um die Lage der Längsachse des Rohres.

Insbesondere bei einem zylinderförmigen Werkzeug ist vorgesehen, dass sich die Strömungskanäle jeweils entlang einer Richtung erstrecken, die windschief zu der Zylinderachse des Werkzeugs verläuft.

Weiterhin ist gemäß einer Ausführungsform des Verfahrens vorgesehen, dass die Strömungskanäle bzw. Rillen im Querschnitt kreissegmentförmig ausgebildet sind. Hierdurch liegen am Übergang bzw. Durchbruch des jeweiligen Strömungskanals zur Außenseite des Werkzeugs jeweils zwei scharfe Schneidkanten vor. Die Rillen können jedoch auch andere Querschnittsformen aufweisen.

Weiterhin ist gemäß einer Ausführungsform des Verfahrens vorgesehen, dass die Außenseite des Werkzeugs eine Oberflächenstruktur aufweist, die zum abrasiven Einwirken auf die Innenseite des Rohres ausgebildet ist, wobei bei dem Bewegen und dem Rotieren des Werkzeugs im Innenraum des Rohres von der Innenseite des Rohres abragende Verunreinigungen des Rohres mit Hilfe der Oberflächenstruktur abgetragen werden.

Weiterhin ist gemäß einer Ausführungsform des Verfahrens vorgesehen, dass das Werkzeug bei dem Bewegen entlang der Längsachse hin und her bewegt wird, indem einander abgewandte Seiten des Werkzeugs im Innenraum des Rohres mit unterschiedlichen Drücken beaufschlagt werden bzw. unterschiedlich mit einem gasförmigen Medium beaufschlagt werden oder indem das magnetische Wechselfeld bzw. die besagte Spule entlang der Längsachse bezüglich des Rohres entsprechend hin und her bewegt wird. Bei der Verwendung eines gasförmigen Mediums (z. B. Druckluft) kann z. B. abwechselnd an einer Öffnung des Rohres ein Unterdruck an den Innenraum des Rohres und an der gegenüberliegenden Öffnung ein Überdruck an den Innenraum des Rohres angelegt werden. Das dazwischen befindliche Werkzeug bewegt sich dann entsprechend zum Unterdruck hin, so dass es im Innenraum hin- und her bewegbar ist.

Weiterhin ist gemäß einer Ausführungsform des Verfahrens vorgesehen, dass das Werkzeug zum Bewegen und Rotieren des Werkzeugs mittels des magnetischen Wechselfeldes zumindest einen Permanentmagneten aufweist. Beispielsweise können zwei Permanentmagnete verwendet werden, die in das Werkzeug eingelassen sein können und sich z. B. jeweils auf beiden Seiten der Rotationsachse parallel zur Rotationsachse erstrecken können, so dass die Rotationsachse zwischen den beiden Permanentmagneten verläuft.

Weiterhin ist gemäß einer Ausführungsform des Verfahrens vorgesehen, dass mittels des Werkzeugs eine entlang der Längsachse variierende Wanddicke des Rohres erzeugt wird, indem das Werkzeug (z. B. mittels der Spule oder entsprechender Druckbeaufschlagung) definiert entlang der Längsachse im Innenraum des Rohres positioniert wird und in der jeweiligen Position rotiert wird.

Weiterhin ist gemäß einer Ausführungsform des Verfahrens vorgesehen, dass das Rohr aus einer Metalllegierung gefertigt ist, insbesondere einer Nickel-Titan-Legierung (z. B. Nitinol) oder einer Cobalt-Chrom-Legierung oder einem Chrom-Nickel Stahl.

Weiterhin ist gemäß einer Ausführungsform des Verfahrens vorgesehen, dass das Rohr einen Innendurchmesser im Bereich von 1,5mm bis 10mm aufweist.

Weiterhin ist gemäß einer Ausführungsform des Verfahrens vorgesehen, dass das Rohr eine Länge von zumindest 1 m, insbesondere von zumindest 1,5 m, insbesondere von zumindest 2m, aufweist. Die Länge des Rohres kann z. B. im Bereich von 1m bis 5m, insbesondere im Bereich von 1m bis 3m, insbesondere im Bereich von 1,5 m bis 2,5 m, liegen.

Weiterhin ist gemäß einer Ausführungsform des Verfahrens vorgesehen, dass es sich bei dem Rohr um einen Rohling für ein Medizinprodukt handelt, insbesondere um einen Rohling für ein medizinisches Implantat, insbesondere um einen Rohling für einen Stent oder einen Träger (z. B. Gerüst) für eine Herzklappe (z. B. TAVI) oder um eine Kanüle für Injektionsnadeln die höchsten Reinheitsanforderungen ausgesetzt ist.

Gemäß einer weiteren Ausführungsform der Erfindung kann vorgesehen sein, dass der mittels des Werkzeugs bearbeitete Rohling zu einem medizinischen Implantat (z. B. Stent oder Scaffold) oder zu einer Komponente (z. B. Stent oder Scaffold) eines medizinischen Implantats weiterverarbeitet wird.

Ein weiterer Aspekt der vorliegenden Erfindung betrifft eine Einrichtung zur Bearbeitung einer Innenseite eines entlang einer Längsachse erstreckten Rohres, wobei die Innenseite einem Innenraum des Rohres zugewandt ist bzw. diesen Innenraum begrenzt, und wobei die Einrichtung zumindest aufweist: ein Werkzeug, das zum Einführen in den Innenraum des Rohres ausgebildet ist, wobei das Werkzeug eine Außenseite aufweist in der eine Vielzahl an Strömungskanälen in Form von Rillen ausgebildet sind, die in einer Umfangsrichtung des Körpers nebeneinander angeordnet sind, und wobei jede Rille an der Außenseite jeweils zwei Schneidkanten ausbildet, die insbesondere zum Abtrennen von Verunreinigungen ausgebildet sind, die von der Innenseite des Rohres abragen.

Gemäß einer Ausführungsform der Einrichtung ist vorgesehen, dass sich die Strömungskanäle bzw. Rillen über eine gesamte Länge des Werkzeugs in axialer Richtung (d. h. entlang der Rotationsachse des Werkzeugs) erstrecken.

Weiterhin ist gemäß einer Ausführungsform der Einrichtung vorgesehen, dass die Einrichtung eine Bewegungserzeugungsvorrichtung aufweist, die dazu konfiguriert ist, das Werkzeug im Innenraum des Rohres entlang der Längsachse des Rohres zu bewegen, insbesondere hin- und her zu bewegen, und gleichzeitig in der Umfangsrichtung des Werkzeuges zu rotieren.

Gemäß einer Ausführungsform der Einrichtung ist diesbezüglich vorgesehen, dass die Bewegungserzeugungsvorrichtung dazu ausgebildet ist, das Werkzeug mit einem Druck bzw. einem gasförmigen Medium zu beaufschlagen, so dass das Werkzeug entlang der Längsachse im Innenraum des Rohres bewegt wird, insbesondere hin- und herbewegt wird, und dabei in der Umfangsrichtung rotiert wird.

Weiterhin ist gemäß einer Ausführungsform der Einrichtung vorgesehen, dass die Einrichtung eine verfahrbare Spule aufweist, die dazu ausgebildet ist, ein magnetisches Wechselfeld am Ort des Werkzeuges zu erzeugen (wenn das Werkzeug im Innenraum des zu bearbeitenden Rohres angeordnet ist), um das Werkzeug entlang der Längsrichtung des Rohres zu bewegen (insbesondere hin- und her zu bewegen) und/oder in der Umfangsrichtung zu rotieren. Bevorzugt weist in dieser Ausgestaltung das Werkzeug noch mindestens einen Permanentmagneten auf.

In dieser Ausgestaltung sorgt die Anordnung des/der Permanentmagneten im Werkzeug, die von außen angelegte Frequenz des magnetischen Wechselfeldes und der Vorschub der verfahrbaren Spule in Längsrichtung des Rohres für die Eigenrotationsgeschwindigkeit (Drehzahl) des Werkzeuges im Rohr und die Geschwindigkeit des Werkzeuges in Längsrichtung. Damit lässt sich vorteilhafterweise in dieser Ausgestaltung der Erfindung die einzelnen Beiträge der sich überlagernden Bewegungskomponenten entlang der Längsachse des zu reinigenden Rohres und entlang der Umfangsrichtung des zu reinigenden Rohres sehr gut und genau steuern.

Weiterhin ist gemäß einer Ausführungsform der Einrichtung vorgesehen, dass die Strömungskanäle entlang der Außenseite des Werkzeugs jeweils einen gekrümmten Verlauf aufweisen (siehe auch oben).

Weiterhin ist gemäß einer Ausführungsform der Einrichtung vorgesehen, dass das Werkzeug kugelförmig ausgebildet ist.

Weiterhin ist gemäß einer Ausführungsform der Einrichtung vorgesehen, dass das Werkzeug Körper zylinderförmig ausgebildet ist.

Weiterhin ist gemäß einer Ausführungsform der Einrichtung vorgesehen, dass sich die Strömungskanäle jeweils entlang einer Richtung erstrecken, die windschief zu einer Zylinderachse des Werkzeugs orientiert ist.

Weiterhin ist gemäß einer Ausführungsform der Einrichtung vorgesehen, dass die Strömungskanäle im Querschnitt kreissegmentförmig ausgebildet sind, so dass insbesondere an der Außenseite des Werkzeuges die besagten Schneidkanten vorliegen.

Weiterhin ist gemäß einer Ausführungsform der Einrichtung vorgesehen, dass die Außenseite des Körpers eine Oberflächenstruktur aufweist, die zum abrasiven Einwirken auf die Innenseite des Rohres ausgebildet ist.

Weiterhin ist gemäß einer Ausführungsform der Einrichtung vorgesehen, dass der Körper zum Bewegen und Rotieren des Körpers mittels des magnetischen Wechselfeldes zumindest einen Permanentmagneten aufweist (siehe auch oben).

Gemäß einem weiteren Aspekt der Erfindung wird ein medizinisches Implantat offenbart, aufweisend zumindest einen Abschnitt eines Rohres, das mittels des erfindungsgemäßen Verfahrens bearbeitet worden ist.

Im Folgenden sollen Ausführungsformen sowie Merkmale und Vorteile der Erfindung anhand der Figuren erläutert werden. Es zeigen:
- Fig. 1: eine perspektivische Ansicht einer Ausführungsform eines erfindungsgemäßen Werkzeugs, das kugelförmig ausgebildet ist;
- Fig. 2: eine perspektivische Ansicht einer weiteren Ausführungsform eines erfindungsgemäßen Werkzeugs, das zylinderförmig ausgebildet ist;
- Fig. 3: ein mögliches Detail des in der Figur 2 gezeigten Werkzeugs;
- Fig. 4: eine schematische Ansicht einer Ausführungsform einer erfindungsgemäßen Einrichtung mit einem Werkzeug, das insbesondere nach Art der in der Figur 1 gezeigten Ausführungsform ausgebildet ist;
- Fig. 5: eine perspektivische Ansicht des in der Figur 2 gezeigten Werkzeugs, das in einem Rohr angeordnet ist, um die Innenseite des Rohres zu bearbeiten, insbesondere zu reinigen; und
- Fig. 6: eine weitere Ausführungsform einer erfindungsgemäßen Einrichtung mit einem Werkzeug nach Art der Figur 2;

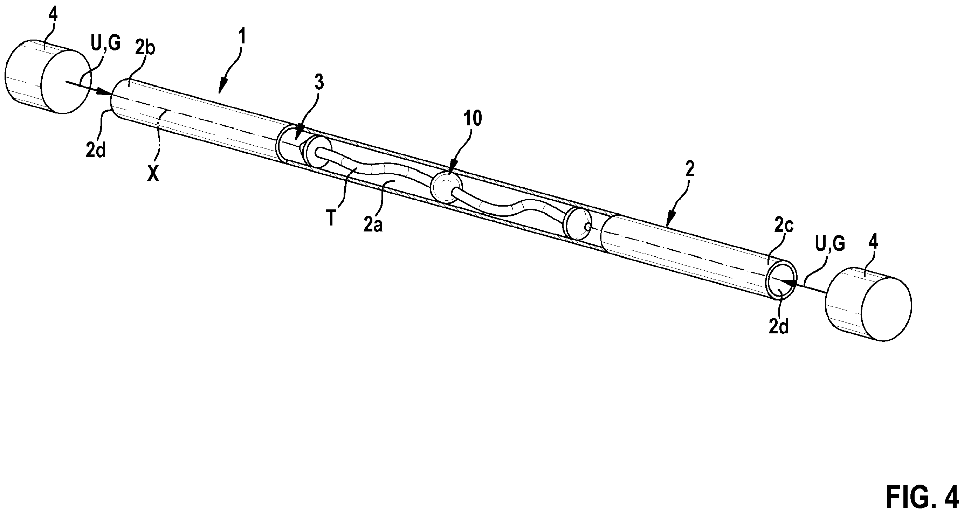

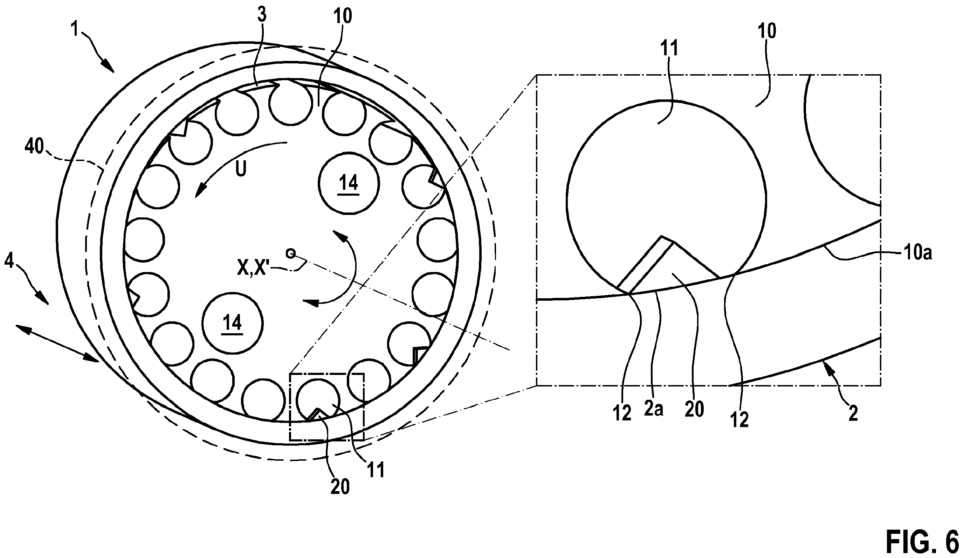

Die vorliegende Erfindung betrifft ein Verfahren zur Bearbeitung einer Innenseite 2a eines entlang einer Längsachse x erstreckten Rohres 2, wobei die Innenseite 2a einem Innenraum 3 des Rohres 2 zugewandt ist, und wobei das Verfahren zumindest die folgenden Schritte aufweist: Einführen eines Werkzeugs 10 in den Innenraum 3 des Rohres 2, wobei das Werkzeug 10 eine Vielzahl an zur Außenseite 10a hin offenen Strömungskanälen 11 in Form von Rillen 11 aufweist, die in einer Umfangsrichtung U des Werkzeugs 10 nebeneinander angeordnet sind, und wobei jede Rille 11 an der Außenseite 10a vorzugsweise jeweils zwei Schneidkanten 12 ausbildet, und Bewegen des Werkzeugs 10 im Innenraum 3 des Rohres 2 entlang der Längsachse x des Rohres 2 sowie gleichzeitiges Rotieren des Werkzeugs 10 in der Umfangrichtung U des Werkzeugs 10, z. B. durch Beaufschlagen des Körpers mit einem Druck bzw. einem gasförmigen Medium (vgl. z. B. Fig. 4) und/oder durch Einwirken auf den Körper mit einem magnetischen Wechselfeld (vgl. z. B. Fig. 6), wobei von der Innenseite des Rohres abragende Verunreinigungen 20 des Rohres 2 oder Materialbereiche 20 des Rohres 2 mittels der Schneidkanten 12 abgetrennt werden. Bei den Verunreinigungen handelt es sich insbesondere um unerwünschte Anhaftungen/Fremdmaterialien des Rohres 2. Es kann sich aber auch um Unebenheiten/Vorsprünge der Innenseite 2a des Rohres 2 bzw. des eigentlichen Rohrmaterials handeln.

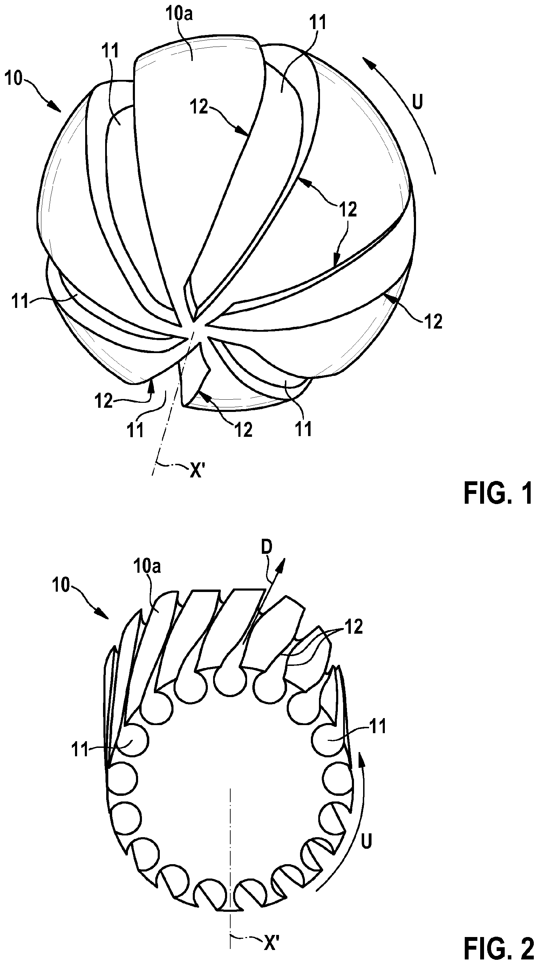

Mögliche Ausgestaltungen des Werkzeuges sind in den Figuren 1 bis 3 sowie 5 und 6 gezeigt.

Die erfindungsgemäße Lösung zielt insbesondere darauf ab, zumindest ein Werkzeug 10, das auch als Innenwerkzeug 10 bezeichnet wird, da es vollständig im Innenraum 3 des Rohres 2 angeordnet wird, die im Rohrinneren 3 befindlichen, haftenden Fremdmaterialien 20 des Rohres 20 abrasiv zu entfernen. Der abrasive Effekt kann dabei sowohl mit dem Prinzip der geometrisch bestimmten Schneide, die hier durch die jeweilige Schneidkanten 12 gebildet ist, als auch mit dem Prinzip der geometrisch unbestimmten Schneide erfolgen. Hierbei kann es sich z. B. um eine Oberflächenstruktur 13 der Außenseite 10a des Werkzeugs 10 handeln.

Das jeweilige Innenwerkzeug 10 weist vorzugsweise einen im Vergleich zum Rohrinnendurchmesser definiert kleineren Außendurchmesser auf. Die äußere Form der Innenwerkzeuge kann sowohl kugelförmig gemäß Figur 1 als auch zylinderförmig gemäß Figur 2 ausgestaltet sein. Die Werkzeuge 10 können des Weiteren z. B. aus einem gebeizten Hartmetall gefertigt sein.

Die Innenwerkzeuge 10 weisen dabei zudem vorzugsweise eine definierte Oberflächenstruktur auf, um die Verunreinigungen 20 der Rohrinnenseite 2a effizient entfernen zu können.

Gemäß einer Ausführungsform ist hierbei gemäß Figuren 1 und 2 eine makroskopische Struktur der äußeren Werkzeugform in Form von scharfkantigen, harten und schneidhaltigen Strömungskanalkanten 12 vorgesehen. Bei den Strömungskanälen bzw. Rillen 11, die z. B. gemäß Figuren 1 und 2 über den Kugelumfang bzw. den Zylinderumfang gekrümmt verlaufen, handelt es sich weiterhin jeweils bevorzugt um bifunktionale Elemente, die beim Anlegen unterschiedlicher Luftdrücke an den Rohrenden bzw. -Öffnungen 2b, 2c des Rohres 2 sowohl eine Rotation in der Umfangsrichtung U des jeweiligen Werkzeuges 10 (bzw. um eine Rotationsachse x' des jeweiligen Werkzeugs 10) als auch eine Bewegung entlang der Längs- bzw. Rohrachse x erzeugen.

Die jeweilige Schneidkante bzw. Strömungskanalkante 12 bewirkt beim Kontakt mit der fremdmaterialbehafteten Innenseite 2a des Rohres 2 einen materialabtragenden Effekt, ohne dass die restliche Rohrinnenwand einem nennenswerten Materialabtrag unterliegt.

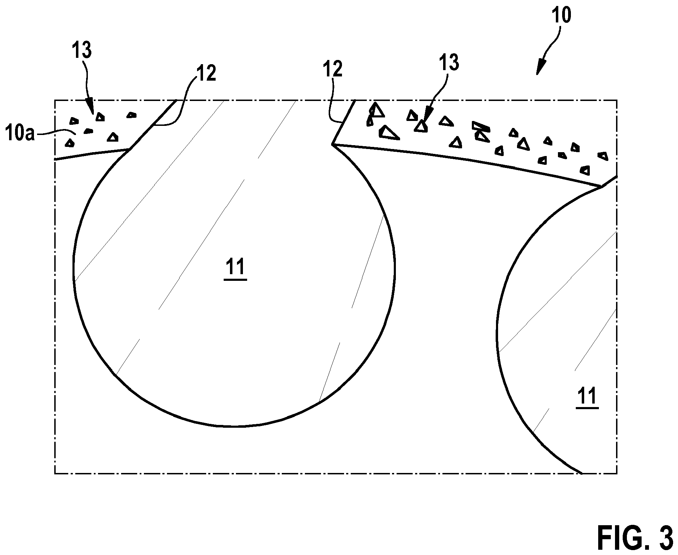

Die Werkzeuge 10 können weiterhin, wie anhand der Figur 3 dargestellt, eine mikroskopisch aufgeraute Oberflächenstruktur 13 der Außenseite 10 (z. B. eine Mikrostruktur) aufweisen, die eine weitere Glättung der Rohrinnenoberflachen bzw. Innenseite 2a ermöglicht. Durch die mikroskopisch aufgeraute Oberfläche wird eine zusätzliche abrasive Wirkung erzielt. Derartige Oberflächen weisen bevorzugt einen Mittelrauwert im Bereich von 0.006 bis 0.2 (bestimmt mit einem Tastschnittgerät) auf. Dadurch wird die Gefahr einer lokalen Aufrauung minimiert und der Forderung nach einer glatten von Mikrorissen und Mikrokerben freien Rohrinnenoberflache erfüllt. Wie in der Figur 3 gezeigt, kann die Oberflächenstruktur 13 durch einen Beizprozess erzeugt werden, der die Hartmetalloberfläche 10a aufraut. Die Oberflächenstruktur 13 kann dann z. B. durch freigelegte Wolframkarbidteilchen gebildet sein. Über den Grad der Freilegung der Wolframkarbidteilchen und die mittlere Größe der Wolframkarbidteilchen wird die Rauheit der Werkzeugoberfläche beeinflusst. Damit kann wiederum die Rauheit der Rohroberfläche variiert werden.

Weiterhin weisen die Strömungskanäle 11, wie z. B. anhand der Figuren 2 und 3 ersichtlich ist, vorzugsweise am Außendurchmesser bzw. an der Außenseite 10a einen Durchbruch auf, der sich beidseitig zu einer (z. B. fräskopfartigen) Schneidkante 12 verjüngt. Die Strömungskanäle 11 können dabei im Querschnitt kreissegmentförmig ausgebildet sein. Die jeweilige Schneide 12 ist dazu ausgebildet, die aus der Rohrinnenoberfläche bzw. aus der Innenseite 2a des Rohres heraustretenden Fremdmaterialien 20 (z. B. Erhebungen, Plateaus, usw.) abzutrennen. Die entstehenden Partikel werden z. B. durch die Druckunterschiede im jeweiligen Strömungskanal 11 abgesaugt (z. B. durch die Beaufschlagung des Werkzeugs 10 mit einem gasförmigen Medium, insbesondere Druckluft).

Die Figur 4 zeigt schematisch eine Trajektorie T der taumelnden Bewegung des kugelförmigen Werkzeuges 10 gemäß Figur 1 im Innenraum 3 des Rohres 2 (z. B. beim Ansaugen mittels Unterdruck von der rechten Rohrseite 2c).

Gemäß einer Ausführungsform der Erfindung wird z. B. ein Rohr 2 aus der Legierung Nitinol nach ASTM F2063 mit einem Außendurchmesser von 7,000 mm und einer Wanddicke von 0,500 mm mittels des erfindungsgemäßen Verfahrens bearbeitet. Der Rohrinnendurchmesser beträgt somit 6,000 mm. Die Rohrinnenoberfläche bzw. Innenseite 2a des Rohres 2 weist unerwünschte plateauartige Erhebungen 20 auf, die z. B. aus Resten von eingebrannten Rohrzughilfsmitteln bestehen. Diese hoch kohlenstoffhaltigen Verunreinigungen 20 weisen eine hohe Adhäsion zur Rohrinnenoberfläche 2a auf und können durch übliche Reinigungsverfahren nicht restlos entfernt werden. In ein solches ca. 2 m langes Rohr 2 wird ein kugelförmiges Werkzeug 10 gemäß Figur 1 eingebracht. Der Außendurchmesser dieser Kugel bzw. dieses Werkzeuges 10 beträgt vorzugsweise 5,950 mm. An beide Rohrenden 2b, 2c des Rohres 2 werden Druckvorrichtungen 4 angebracht (vgl. Figur 4), die eine Bewegungserzeugungsvorrichtung bilden und dazu ausgebildet sind, alternierend immer ein Rohrende 2b, 2c mit einem gasförmigen Medium G in Form von Druckluft und das jeweils andere Ende 2c, 2b mit einem Vakuum bzw. Unterdruck U zu beaufschlagen. An beiden Rohrenden 2b, 2c befinden sich vorzugsweise flexible und luftdurchlässige netzförmige Gitter (Filter) 2d, die z. B. aus einem Kunststoff (Maschenweite ca. 0,5 mm) gefertigt sein können. Diese verhindern das unbeabsichtigte Heraustreten des kugelförmigen Werkzeuges 10 aus dem Rohrinneren 3.

Die Beaufschlagung mit unterschiedlichen Drücken mittels der Bewegungserzeugungsvorrichtung bzw. Druckvorrichtungen 4 führt zu Hin- und Herbewegungen der Kugel 10. Die Strömungskanäle 11 bewirken dabei eine gleichzeitige Rotation der Kugel 10. Beim Auftreffen auf Unebenheiten/Verunreinigungen 20 scheren die scharfkantigen Strömungskanal- bzw. Schneidkanten 12 die Unebenheiten bzw. Verunreinigungen 20 ab und die abgeriebenen Partikel werden über die Strömungskanäle 11 zu den Rohrenden 2b, 2c abgesaugt. Die Maschenweite der Filter 2d ist insbesondere so gewählt, dass diese Partikel ohne Widerstand aus dem Rohrinneren 3 abgesaugt werden können, die Kugel bzw. das Werkzeug 10 aber darin verbleibt. Die Frequenz bzw. die Geschwindigkeit mit der sich die Kugel bzw. das Werkzeug 10 im Innenraum 3 des Rohres 2 bewegt, kann über die Frequenz der Druck-/Vakuumbeaufschlagung G, U eingestellt werden. Bei einer Geschwindigkeit von ca. 1 m/s bis 5 m/s in Richtung der Längsachse x des Rohres 2 rotiert das kugelförmige Werkzeug 10 mit mehreren Umdrehungen pro Sekunde um seine Rotationsachse x'. Nach einer Verweildauer der Kugel 10 im Rohr 2 von ca. 1 min ist sichergestellt, dass jeder Punkt im Rohr 2 mindestens 3 x in Kontakt mit der Kugel 10 kam. Danach liegt die Rohrinnenoberflache bzw. Innenseite 2a des Rohres 2 gereinigt und frei von Kontaminationen durch Fremdmaterial vor. Nach den sich anschließenden Prozessen (Laserschneiden, Beizen, Elektropolieren) zeichnen sich derart hergestellte vaskulare Implantate durch fehlerfreie Innenoberflachen aus. Dies führt zu einer erhöhten Langzeitermüdungsbeständigkeit und letztendlich zu einer erhöhten Produktsicherheit.

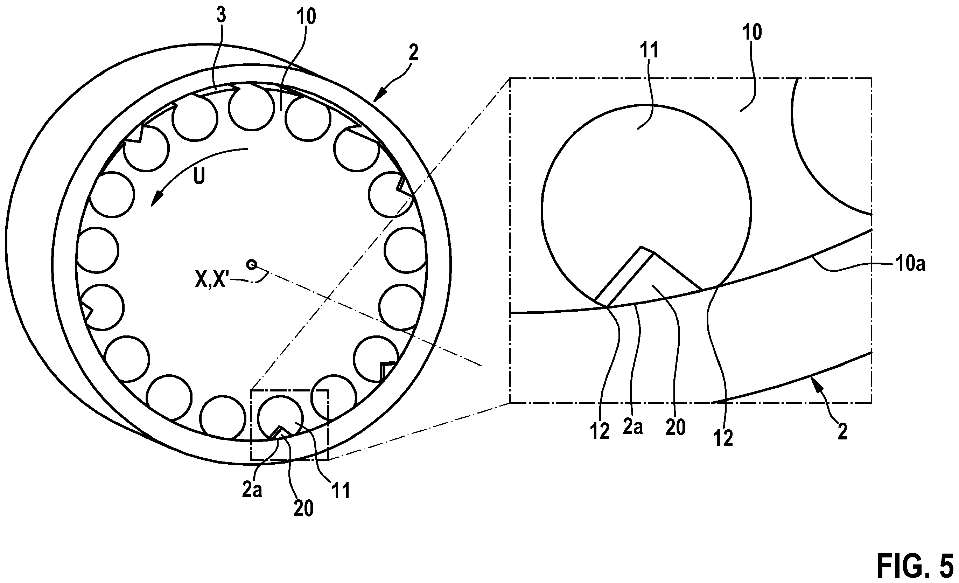

Gemäß einer weiteren Ausführungsform (vgl. z. B. Figur 6) wird ein Rohr aus der Cobalt-Chromlegierung L-605 nach ASTM F90 mit dem erfindungsgemäßen Verfahren bearbeitet. Ein solches Rohr 2 weist z. B. einen Außendurchmesser von 1,800 mm und eine Wanddicke von z. B. 0,090 mm auf. Der Rohrinnendurchmesser beträgt somit 1,620 mm. Die Innenseite 2a des Rohres 2 weist wiederum unerwünschte plateauartige Erhebungen 20 auf, die aus Resten von eingebrannten Rohrzughilfsmitteln, Reinigungslösungen und Materialien des Innenwerkzeuges (Mandrel) bestehen. Dies sind beispielsweise Verbindungen aus Kohlenstoff, Eisen und Chlor. Hinzu kommen noch Elemente aus der Mandreloberfläche wie Zink und Bestandteile des Schmierstoffs wie Molybdän, Schwefel und Phosphor. Ebenfalls möglich sind noch Reste von vorangegangenen Rohrreinigungsverfahren wie Siliziumkarbid oder Aluminiumoxid. Diese teilweise eingebrannten Verunreinigungen 20 haben eine hohe Adhäsion zur Innenseite 2a des Rohres 2 und können durch übliche Reinigungsverfahren wie Beizen oder Sandstrahlen nicht restlos entfernt werden. In ein solches ca. 2 m langes Rohr 2 wird ein zylinderförmiges Werkzeug 10 nach Figuren 2 und 3 eingebracht, das dann, wie in den Figuren 4 und 5 gezeigt, im Innenraum 3 des Rohres 2 angeordnet ist.

Der Außendurchmesser dieses strukturierten Zylinders bzw. Werkzeugs 10 aus Hartmetall beträgt vorzugsweise 1,550 mm. Die beiden Rohrenden des Rohres 2 werden vorzugsweise in einer Vorrichtung derart eingespannt, dass durch mitlaufende Lünetten eine übermäßige Rohrverbiegung weitgehend vermieden wird. Um den Außenseite des Rohres 2 herum wird eine mit Wechselstrom betriebene Spule 40 platziert, die in der Figur 6 schematisch durch einen gestrichelten Kreis angedeutet ist. Durch Integration einer oder zweier z. B. stangenförmiger Permanentmagneten im Inneren des zylinderförmigen Werkzeuges 10 kommt es bei Inbetriebnahme der Spule 40 zu einer Rotationsbewegung des zylindrischen Werkzeugs 10 um die Rotations- bzw. Zylinderachse x' des Werkzeugs 10. Die Umdrehungsgeschwindigkeit kann dabei durch die elektrischen Parameter, mit denen die Spule 40 betrieben wird, variiert werden. Sowohl die Schneidkanten 12 als auch die auf der Außenseite 10a Werkzeugs 10 befindliche Oberflächenstruktur 13 bewirken ein von Fremdmaterial freies sowie gleichmäßig mikrostrukturiertes Rohrinneres 3. Dieser nunmehr vorliegende Oberflächenzustand der Innenseite 2a des Rohres 2 führt mit Vorteil zu einem gleichmäßigeren Materialabtrag, der nach dem Laserschneiden nachfolgenden Prozessschritte wie Beizen und Elektroplieren. Durch eine Längsverschiebung der Spule 40 in Richtung der Längsachse x des Rohres wird die gesamte Rohrinnenoberfläche bzw. Innenseite 2a des Rohres 2 behandelt.

Weiterhin bietet die Ausführungsform nach Figur 6 die Möglichkeit, den Materialabtrag durch eine spezifische Positionierung der Spule 40 nicht über die gesamte Rohrlänge vorzunehmen. Somit eröffnet sich die Möglichkeit der abschnittsweisen Reduktion der Wanddicke von der Innenseite 2a des Rohres 2 her. Das so hergestellte, innenstrukturierte Rohr 2 ermöglicht die Fertigung von Stentdesigns mit über die Stentlänge variablen Wandstärken.

Die Möglichkeit der erfindungsgemäßen Innenbearbeitung von Rohren eröffnet folgende Vorteile. Zunächst führt eine Verbesserung der Halbzeugqualität zu signifikanten Senkungen der Ausschussquote bei den Endprodukten. Ferner sinkt mit Vorteil der technologische Aufwand bei den Nachbearbeitungsprozessen (z. B. Sandstrahlen lasergeschnittener Stents). Die Vereinheitlichung der Rohrinnenoberflächen ermöglicht weiterhin einen gleichmäßigeren Materialabtrag der möglichen nachfolgenden, chemischen und elektrochemischen Prozesse. Dies wiederum lässt eine Erweiterung der Prozessgrenzen zu. Dadurch können extrem dünnwandige Stents für neue Anwendungsgebiete (z. B. cranial) hergestellt werden.

Die einheitlichere Ausgangsoberfläche führt ebenso zu einer größeren Uniformität der finalen Stentinnenoberflächen. Eine höhere Ermüdungsfestigkeit verbunden mit einer größeren Medizinproduktsicherheit ist die Folge.

Bei längerer Bearbeitungszeit können zudem Rohrwanddicken erzeugt werden, die mittels konventioneller Rohrzugtechnologien nicht mehr herstellbar sind.

Weiterhin sind insbesondere bei Verwendung des zylinderförmigen Werkzeuges 10 lokal begrenzte Veränderungen des Rohrinnendurchmessers möglich. Dies eröffnet die Möglichkeit zur Herstellung wanddickenvariabler Stents.

## Patentansprüche

1. Verfahren zur Bearbeitung einer Innenseite (2a) eines entlang einer Längsachse (x) erstreckten Rohres (2), wobei die Innenseite (2a) einen Innenraum (3) des Rohres (2) zugewandt ist, und wobei das Verfahren zumindest die folgenden Schritte aufweist:
- Einführen eines Werkzeugs (10) in den Innenraum (3) des Rohres (2), wobei das Werkzeug (1) eine Außenseite (10a) aufweist, in der eine Vielzahl an Strömungskanälen (11) in Form von Rillen ausgebildet ist, die in einer Umfangsrichtung (U) des Werkzeugs (10) nebeneinander angeordnet sind, und wobei jede Rille (11) an der Außenseite (10a) jeweils mindestens eine, bevorzugt zwei, Schneidkante(n) (12) ausbildet, und
- Bewegen des Werkzeugs (10) im Innenraum (3) des Rohres (2) entlang der Längsachse (x) des Rohres (2) sowie gleichzeitiges Rotieren des Werkzeugs (10) in der Umfangrichtung (U) des Werkzeugs (10) durch Beaufschlagen des Werkzeugs (10) mit einem gasförmigen Medium (G) und/oder durch Einwirken auf das Werkzeug mit einem magnetischen Wechselfeld , wobei von der Innenseite (2a) des Rohres (2) abragende Verunreinigungen (20) des Rohres (2) mittels der Schneidkanten (12) abgetrennt werden.

2. Verfahren nach Anspruch 1, wobei die Strömungskanäle (11) entlang der Außenseite (10a) jeweils einen gekrümmten Verlauf aufweisen.

3. Verfahren nach Anspruch 1 oder 2, wobei das Werkzeug (10) kugelförmig oder ellipsoidal, insbesondere als Rotationsellipsoid, ausgebildet ist.

4. Verfahren nach Anspruch 1 oder 2, wobei das Werkzeug (10) zylinderförmig ausgebildet ist.

5. Verfahren nach Anspruch 4, wobei sich die Strömungskanäle (11) jeweils entlang einer Richtung (D) erstrecken, die windschief zu einer Zylinderachse (x') oder Rotationsachse (x') des Werkzeugs (10) ist.

6. Verfahren nach einem der vorhergehenden Ansprüche, wobei die Strömungskanäle (11) im Querschnitt kreissegmentförmig ausgebildet sind.

7. Verfahren nach einem der vorhergehenden Ansprüche, wobei die Außenseite (10a) des Werkzeugs (10) eine Oberflächenstruktur (13) aufweist, die zum abrasiven Einwirken auf die Innenseite (2a) des Rohres (2) ausgebildet ist, wobei bei dem Bewegen und dem Rotieren des Werkzeugs (10) im Innenraum (3) des Rohres (2) von der Innenseite (2a) des Rohres (2) abragende Verunreinigungen (20) des Rohres (2) mittels der Oberflächenstruktur (13) abgetragen werden.

8. Verfahren nach einem der vorhergehenden Ansprüche, wobei das Werkzeug (10) bei dem Bewegen entlang der Längsachse (x) hin und her bewegt wird, indem einander abgewandte Seiten (10b, 10c) des Werkzeugs (10) alternierend mit einem gasförmigen Medium (G) beaufschlagt werden oder indem das magnetische Wechselfeld entlang der Längsachse (x) bezüglich des Rohres (2) entsprechend hin und her bewegt wird.

9. Verfahren nach einem der vorhergehenden Ansprüche, wobei das Werkzeug (10) zum Bewegen und Rotieren des Werkzeugs (10) mittels des magnetischen Wechselfeldes zumindest einen Permanentmagneten (14) aufweist.

10. Verfahren nach einem der vorhergehenden Ansprüche, wobei mittels des Werkzeugs (10) eine entlang der Längsachse (x) variierende Wanddicke des Rohres (2) erzeugt wird, indem das Werkzeug (10) definiert entlang der Längsachse (x) bezüglich des Rohres (2) positioniert wird und in der jeweiligen Position rotiert wird.

11. Verfahren nach einem der vorhergehenden Ansprüche, wobei es sich bei dem Rohr (2) um einen Rohling für ein Medizinprodukt handelt.

12. Einrichtung (1) zur Bearbeitung einer Innenseite (2a) eines entlang einer Längsachse (x) erstreckten Rohres (2), wobei die Innenseite (2a) einem Innenraum (3) des Rohres (2) zugewandt ist, mit: einem Werkzeug (1), das zum Einführen in den Innenraum (3) des Rohres (2) ausgebildet ist, wobei das Werkzeug (10) eine Außenseite (10a) aufweist, in der eine Vielzahl an Strömungskanälen (11) in Form von Rillen ausgebildet ist, die in einer Umfangsrichtung (U) des Werkzeugs (10) nebeneinander angeordnet sind, und wobei jede Rille (11) an der Außenseite (10a) jeweils zwei Schneidkanten (12) ausbildet.

13. Einrichtung nach Anspruch 12, wobei die Einrichtung (1) eine Bewegungserzeugungsvorrichtung (4) zum Bewegen des Werkzeugs (10) im Innenraum (3) des Rohres (2) entlang der Längsachse (x) des Rohres (2) sowie zum gleichzeitigen Rotieren des Werkzeugs (10) in der Umfangsrichtung (U) des Werkzeugs (10) aufweist.

14. Einrichtung nach Anspruch 13, wobei die Bewegungserzeugungsvorrichtung (4) dazu ausgebildet ist, das Werkzeug (10) mit einem gasförmigen Medium (G) zu beaufschlagen, so dass das Werkzeug (10) entlang der Längsachse (x) im Innenraum (3) des Rohres (2) bewegt wird und dabei in der Umfangsrichtung (U) rotiert wird; oder wobei die Bewegungserzeugungsvorrichtung (4) eine verfahrbare Spule (40) aufweist, die dazu ausgebildet ist, ein magnetisches Wechselfeld am Ort des Werkzeugs (10) zu erzeugen, um das Werkzeug (10) entlang der Längsachse (x) zu bewegen und/oder in der Umfangsrichtung (U) zu rotieren.

15. Einrichtung nach einem der Ansprüche 12 bis 14, wobei die Strömungskanäle (11) entlang der Außenseite (10a) des Werkzeugs (10) jeweils einen gekrümmten Verlauf aufweisen.
